# EUROPEAN PATENT SPECIFICATION

(11) **EP 1 607 005 B1**
(45) Date of publication and mention of the grant of the patent: **08.10.2008**
(21) Application number: 05020850.3
(22) Date of filing: 09.12.2002
(51) Int. Cl.: A23L 2/00, A61K 33/00

(54) **The effect of a buffering agent on acidogenesis of plaque**
Die Wirkung eines Puffers auf die Säurebildung durch Plaque
L'effect d'un agent tampon à l'acidification de plaque

(30) Priority: 11.12.2001 US 340769 P; 27.11.2002 US 306970
(43) Date of publication of application: 21.12.2005
(62) Divisional of application: 02258485.8
(73) Proprietor: Wang, Xiao Bing, Sparks MD 21152 (US); Morisawa, Shinkatsu, Kyoto 610-1101 (JP)
(72) Inventor: Wang, Xiao Bing, Sparks MD 21152 (US); Morisawa, Shinkatsu, Kyoto 610-1101 (JP)
(74) Representative: Lipscombe, Martin John

(56) References cited:
- WO-A-00/33757
- US-A- 5 938 915
- US-A- 5 993 785
- US-B1- 6 251 259

## Description

### Field of the Invention

This invention provides a Trim water formulation that minimizes acidification from forming inside the mouth to treat dental caries. The Trim water may be provided in the form of mouthwash formulations to treat dental caries.

### Related Art

Dental caries (tooth decay) is one of the most common oral diseases. Dental caries often appears as a white chalky area on the tooth enamel. This occurs in teeth where microbial plaque exists. Fermentable carbohydrates such as sugars in the diet are metabolized to acids such as lactic acid by plaque bacteria causing a pH change at the tooth surface. If the pH is sufficiently acidic and is not neutralized, the tooth, which is comprised mainly of calcium phosphate crystals such as hydroxyapatite will dissolve or decalcify producing a carious lesion. This area of the tooth then softens to later break down the structure of the tooth to form a cavity. If the tooth is not treated in the early stage, it can progress towards the pulp and require extensive treatment to save the tooth.

A diet with a high concentration of sugar increases the risk of tooth decay. After a single sugar challenge, some surface decalcification will take place, but the damage will be reversed (recalcification) if the acid is neutralized within a short period of time after the sugar challenge (within 40 to 60 minutes). This neutralization, or buffering, takes place naturally by ammonia-producing bacteria in plaque. If sugar challenges are frequent, especially with children that frequently eat sweet snacks during the day, neutralization of plaque pH and recalcification will not take place. Cavitation of the tooth will result if the latter process continues for a few months. Frequent sugar challenges also cause the microbial composition of plaque to shift toward one which is more acidogenic and aciduric. A pH at or below 5.5 is generally accepted as critical for decalcification.

Limiting the dietary intake of sugar can reduce the chance of dental caries from occurring. This is, however, very difficult for most children who crave sugar. Another approach would be enhancement of plaque neutralization after sugar challenges. This has been attempted experimentally by having subjects chew sugar-free chewing gum after sweet snacks, which theoretically stimulates saliva flow and thereby increases salivary bicarbonate concentrations, the main buffer in saliva. The increased saliva flow and the chewing action also helps clear the oral sugar concentrations. Reduction of dental caries has been reported using this technique (Scheinin, Acta Odont Scan 33:267, *1975*). However, chewing a gum after a sugar intake may not be appropriate at times and the gum may need to be chewed for some time to increase the saliva flow.

US 6 251 259 discloses apparatus for producing a reducing electrolysed water having a negative oxidation-reduction potential (ORP). The apparatus produces, in one example, electrolysed water wilth an ORP of -550 mV and a pH of 9.03. US 5 938 915 discloses electrolysed water having an ORP in the range -150 mV to 0 mV and a pH between 8 and 9.5; and use of such water for medical treatment.

### SUMMARY OF THE INVENTION

This invention provides a Trim water mouthwash formulation as defined in claim 1 of the claims appended hereto to prevent or treat dental caries by preventing the acidification solution from forming inside the mouth. Trim water (alkaline) is used as a post-sugar snack mouthwash rinse that neutralizes plaque acids and enhances recalcification of teeth. As such, rinsing with Trim water substantially prevents dental caries from occurring in teeth.

The Trim water may be placed into a cariostatic formulation to be administered near the tooth. The Trim water may be acidic or alkaline. The time of administration may be for about one minute near the location of the teeth. For example, a mouthwash rinse may be rinsed for about 1 minute. Preferably, the mouth rinse of the invention results in the maintenance of pH above 5.5 near the tooth.

These and other objects of the invention will be more fully understood from the following description of the invention, the referenced drawings attached hereto and the claims appended hereto.

### BRIEF DESCRIPTION OF THE DRAWINGS

The present invention will become more fully understood from the detailed description given below, and the accompanying drawings which are given by way of illustration only, and thus are not limitative of the present invention, and wherein;

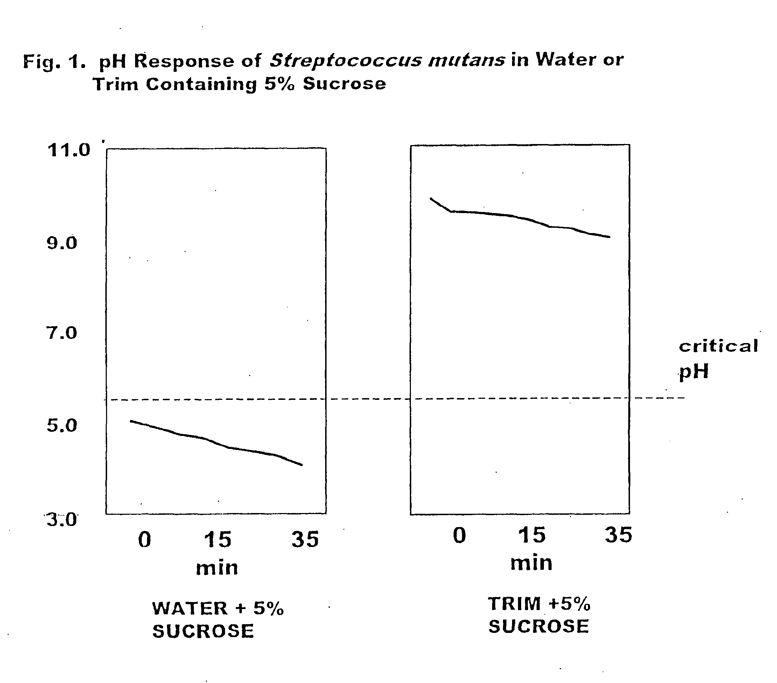

Figure 1 shows the pH response of *Streptococcus mutans* in plain water or Trim water containing 5% sucrose.
Figure 2 shows the pH response of tooth #26 after a one-minute rinse with water.
Figure 3 shows the pH response of tooth #26 after a one-minute rinse with Trim water.
Figure 4 shows the pH response of tooth #27 after a one-minute rinse with water.
Figure 5 shows the pH response of tooth #27 after a one-minute rinse with Trim water.
Figure 6 shows the pH response of tooth #28 after a one-minute rinse with water.
Figure 7 shows the pH response of tooth #28 after a one-minute rinse with Trim water.
Figure 8 shows the pH response of tooth #29 after a one-minute rinse with water.
Figure 9 shows the pH response of tooth #29 after a one-minute rinse with Trim water.
Figure 10 shows the pH response of tooth #26 in Trim water containing 5% sucrose.
Figure 11 shows the pH response of tooth #27 in Trim water containing 5% sucrose.
Figure 12 shows the pH response of tooth #28 in Trim water containing 5% sucrose.
Figure 13 shows the pH response of tooth #29 in Trim water containing 5% sucrose.
Figures 14A to 14L shows sequential radiography of teeth exposed to *Streptococcus mutans* and *Lactobacillus casei.*

### DETAILED DESCRIPTION OF THE PREFERRED EMBODIMENTS

As described in this application, the term "Trim" and "Trim water" are used interchangeably. Furthermore, Trim water is described in United States Patent No. 5,938,915.

One way the Trim water may be made comprises the steps of providing an electrolytic water treatment apparatus including a cathode chamber with a cathode and an anode chamber with an anode, wherein the chambers are separated by a diaphragm, and further introducing raw water into the cathode chamber and the anode chamber, applying a current within a range of about 0.16 mA/cm² to about 3.2 mA/cm² per each pair of electrodes and a diaphragm across the cathode and anode for about 0.5 seconds to about 5 seconds for electrolyzing the raw water, and then extracting the electrolyzed water from the cathode chamber, thereby obtaining the Trim water.

In certain instances, the current range may be within a range of about 0.224 mA/cm² to about 1.6 mA/cm². In another embodiment, the extracted electrolyzed water may be boiled or further filtered. By Trim water, boiled and filtered water are included so long as the water has passed through the current as discussed above.

Experiments to evaluate the buffering potential of Trim water and three experiments using *in vitro* cariogenic plaque are described below. Experiments were designed to test anti-cariogenic potential of Trim as a 1) post sugar rinse, 2) base for a sweet beverage (not according to the invention), and 3) cariostatic agent for active carious lesions.

Since *in vivo* rinsing exposes Trim water to human saliva that contains a bicarbonate buffering system, the pH of Trim water was measured after mouth rinsing for different periods of time (Table 1). A 3-minute rinse with either acidic or alkaline Trim water showed salivary buffering to change the pH from 11.2 (alkaline) or 2.94 (acidic) to between 6 and 7. With shorter rinse of up to one minute, alkaline Trim remained above pH 9 even when sucrose was present.

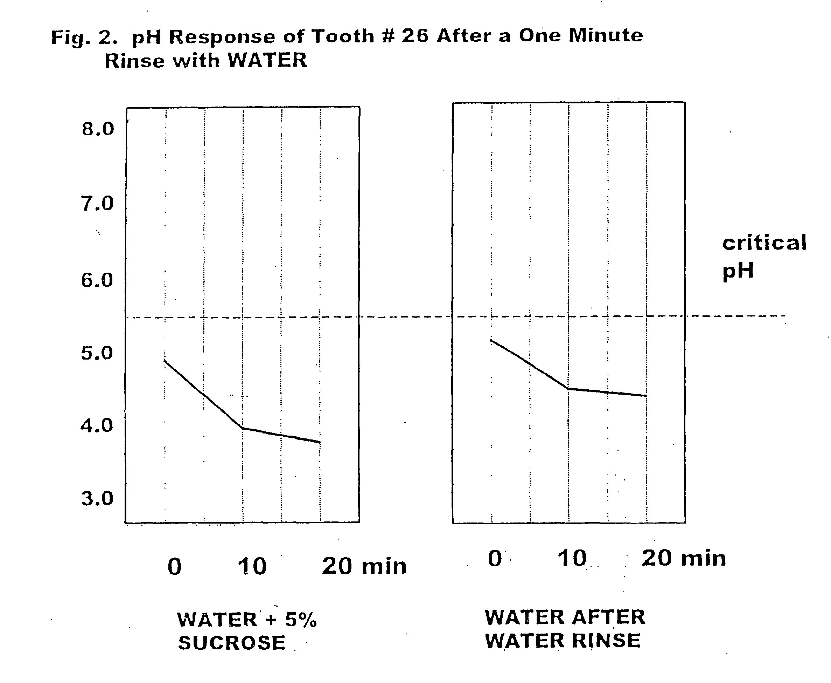

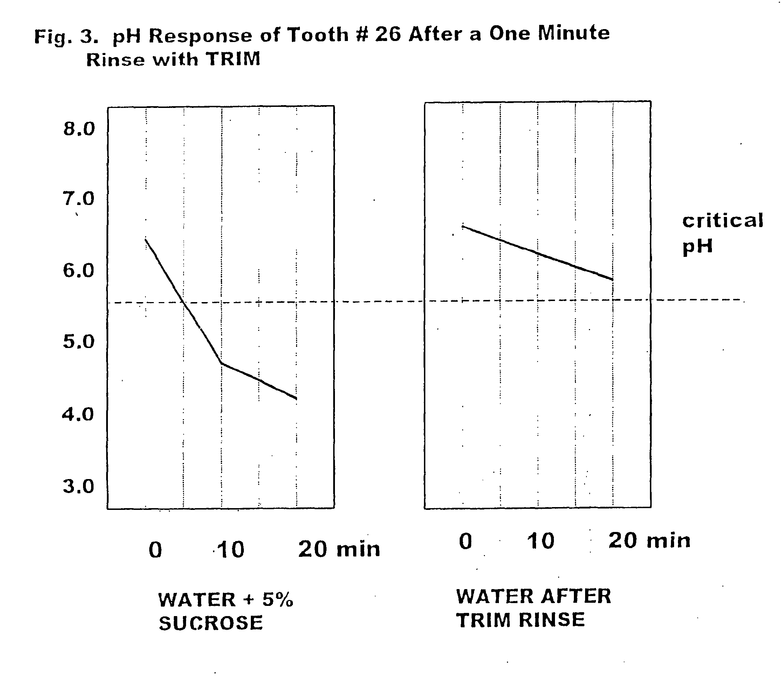

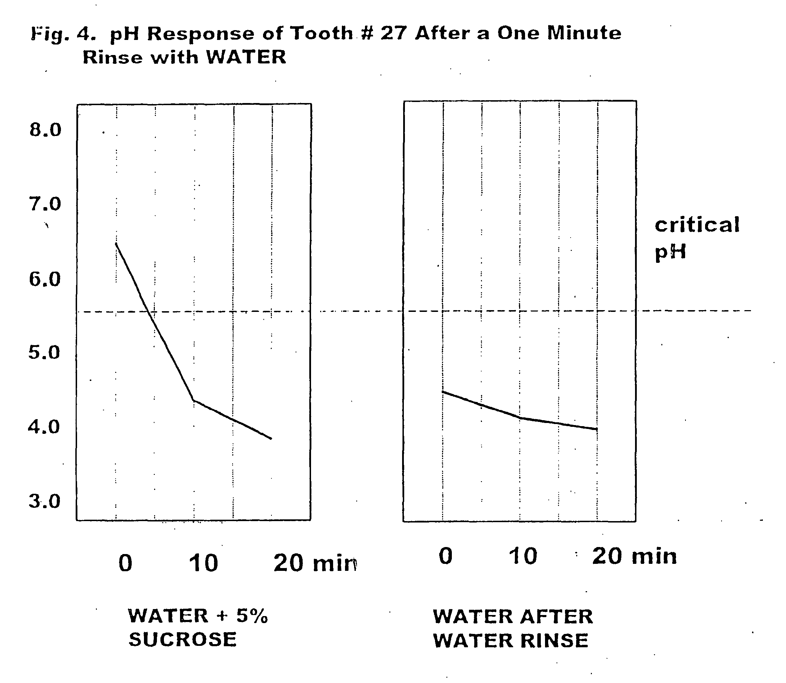

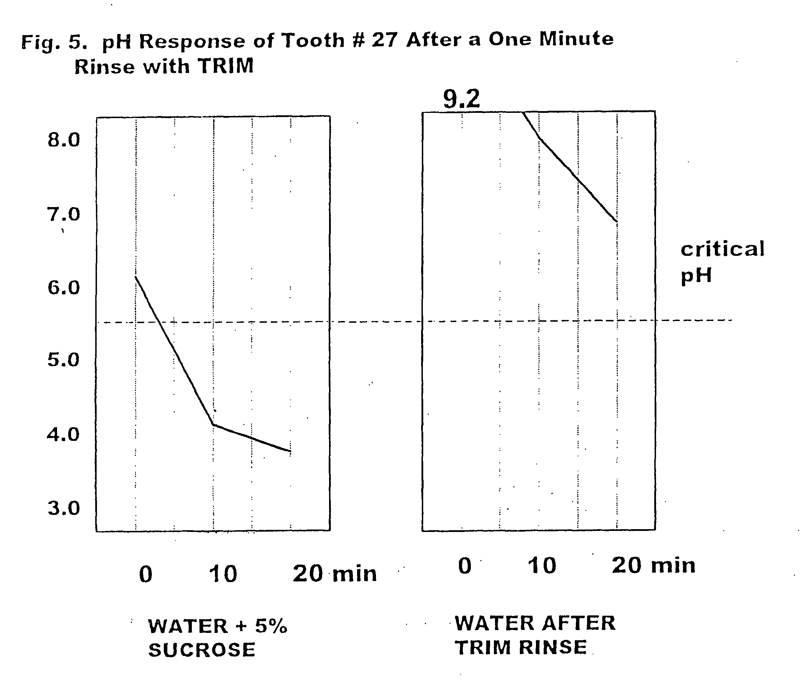

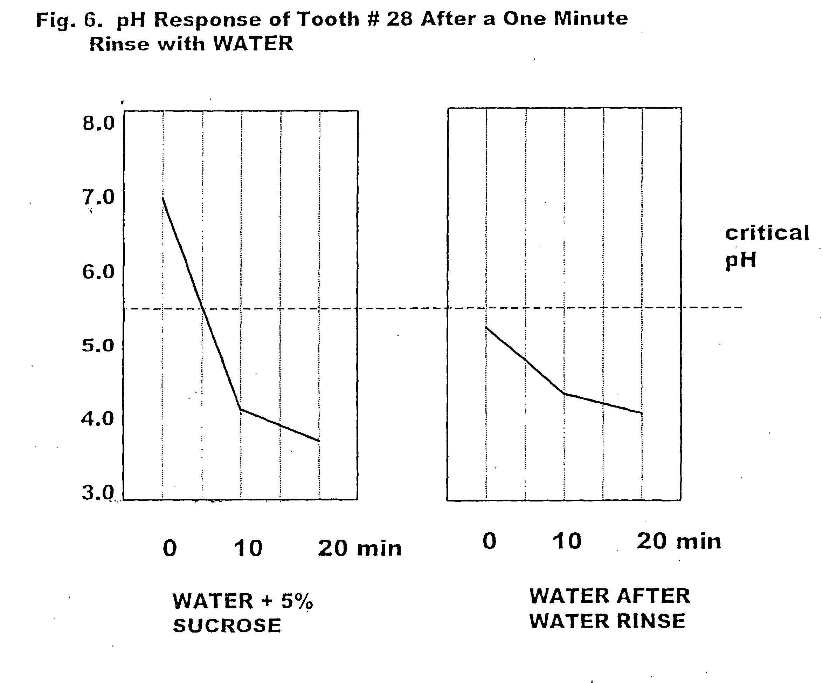

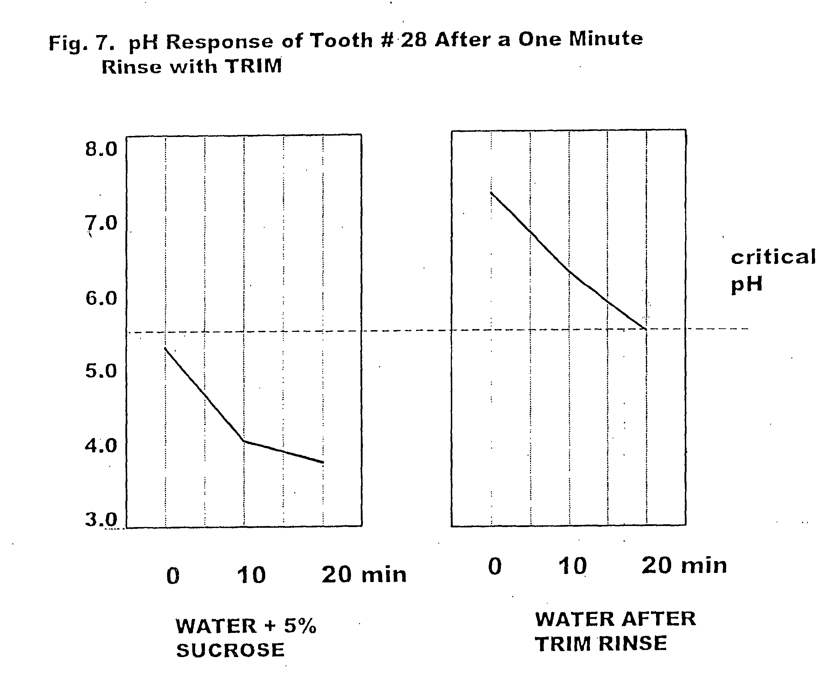

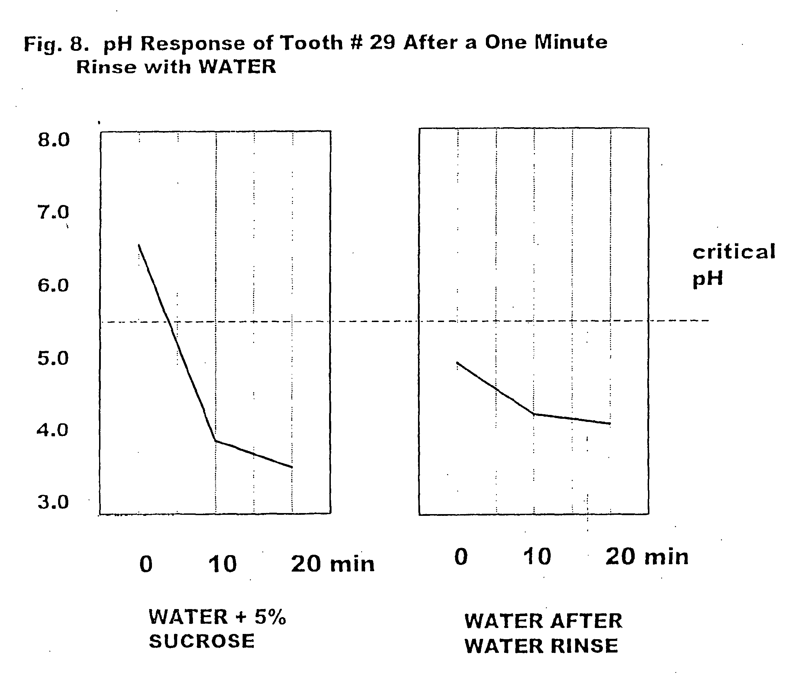

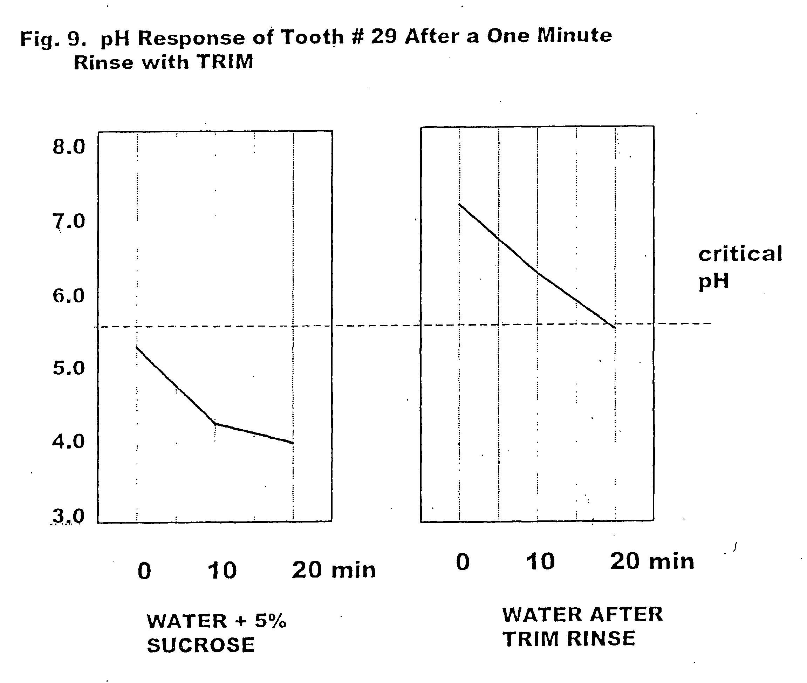

Using caries model experiments, the following advantages of the inventive method are seen.
1. A 1-minute rinse with alkaline Trim water produced a rapid buffering of sucrose-exposed experimental cariogenic dental plaques. The pH remained above 5.5, which is the generally accepted pH when the enamel starts to decalcify, for at least 25 minutes. Normally, most sugar-containing snacks or beverages become cleared from the mouth within 25 minutes. Water rinsing did not provide an adequate anti-acid effect. (Figures 2-9)
2. Compared with water containing sucrose, Trim plus sucrose kept the pH above 5.5 for at least 25 minutes. When Trim plus sucrose was cleared from the plaques by a 1-minute water rinse, the pH continued to remain above 5.5 for an additional 25 minutes. Water plus sucrose failed to maintain the plaque pH within the protective range.
3. Trim rinsing did not greatly alter the pH within active experimental carious lesions which remained fairly constant at approximately pH 4.

Thus, frequent oral exposure to Trim in the form of a mouth rinse may have caries-preventative effects, particularly if employed immediately after a sugar challenge.

This should provide a non-cariogenic, and may also produce an anti-caries effect since an alkaline pH enhances recalcification of teeth.

In addition, use of Trim to arrest active carious lesions or prevent acidogenesis within the lesions does not seem feasible.

The following examples are offered by way of illustration of the present invention, and not by way of limitation.

### EXAMPLES

### Example 1. Effect of Trim Water on Streptococcus mutans Exposed to Sucrose

The purpose of this experiment is to determine if Trim can reduce acidogenicity of *S. mutans*.

As a control, washed cells of *Streptococcus mutans* (GEM), the primary pathogen in human dental caries, was incubated in water containing 5% sucrose for 35 minutes. The pH changes were monitored with a pH meter. The same number of cells was also incubated in Trim water containing 5% sucrose for 35 minutes and the pH monitored.

Water containing 5% sucrose showed a decrease in pH from 5.02 to 4.06 during 35 minutes exposed to *S. mutans*. With Trim water containing 5% sucrose the pH decreased from 10.4 to 9.15. The latter pH range was well within the safe range for tooth integrity. (Decalcification usually takes place below pH 5.5)

### Example 2. In vivo Mouth Rinse with Trim or Trim Plus Sucrose

The purpose of this experiment is 1) to test the effect of oral exposure on the pH of Trim with and without sucrose and 2) to determine if Trim will buffer acidogenesis of salivary bacteria for a prolonged period of time.

Trim containing 10% sucrose was rinsed *in vivo* for 3 minutes and then expectorated into a container, which was incubated at 37°C for 110 minutes. The pH was monitored with a pH meter. A control solution with water containing 10% sucrose was examined similarly.

Trim was rinsed *in vivo* for 5, 10, 15 and 30 seconds. After each rinse it was expectorated into a container and the pH was measured. The same experiment was repeated with water.

Trim containing 10% sucrose was rinsed *in vivo* for 5, 15, 30 and 60 seconds. After each rinse it was expectorated into a container and the pH was measured. The same experiment was repeated with water.

A 3-minute rinse with Trim containing 10% sucrose *in vivo* caused a pH drop from 10.33 (original pH of Trim plus sucrose) to 6.25. After incubation of the expectorated rinse containing saliva for 110 minutes at 37°C, the pH decreased to 5.59.

The same experiment with water plus 10% sucrose resulted in a pH change from 5.61 to 6.45 after a 3-minute rinse *in vivo*. During the 110-minute incubation of the expectorated rinse, the pH dropped from 6.45 to 5.95.

The same experiment repeated with acidic Trim changed from a pH of 2.94 to 5.45 after a 3 minute rinse and to 5.53 after a 110 minute incubation of the expectorated mouthrinse.

These data show strong buffering potential of saliva when Trim is kept in the mouth for 3 minutes. When shorter rinses (up to 1 minute) with Trim were employed, the pH remained high. (Table 1)

**Table 1. In Vivo Rinses with 20ml of Trim or Water Solutions**

| Rinse time | Trim+10% sucrose pH | Water+10% sucrose pH | Acidic Trim+10% sucrose pH | Trim pH | Water pH |
|---|---|---|---|---|---|
| Original | 10.33 | 7.38 | 2.94 | 11.20 | 6.96 |
| 5 sec | 9.54 | 6.62 | | 10.34 | 6.87 |
| 10sec | | | | 10.24 | 6.78 |
| 15 sec | (6.98) | 6.36 | | 9.81 | 6.70 |
| 30 sec | 9.04 | 6.19 | | 9.27 | 6.63 |
| 60 sec | 7.33 | 6.06 | | | |
| 3 min | 6.51 | 6.25 | 5.45 | | |
| Post | | | | | |
| Rinse | | | | | |
| 5 min | 6.45 | 6.21 | 5.45 | | |
| 10 min | 6.53 | 6.30 | 5.46 | | |
| 15min | 6.49 | 6.27 | 5.48 | | |
| 20min | 6.55 | 6.31 | 5.49 | | |
| 20min | 6.55 | 6.31 | 5.49 | | |
| 35min | 6.48 | 6.25 | 5.51 | | |
| 50min | 6.36 | 6.19 | 5.52 | | |
| 110min | 5.95 | 5.59 | 5.53 | | |

### Example 3. In Vitro Dental Caries Model

According to a Dentinal Caries Model System (Minah, Pediat. Dent 20:345,1998), crowns of extracted intact primary teeth were mounted in acrylic bases shaped to fit a holding jig in a digital radiograph apparatus, which permitted accurate positioning for sequential exposures. After enamel was covered with a layer of cold-cure acrylic, the dentin was exposed by circular openings 1.0 mm in diameter made with a # 330 rotating dental bur. Dental caries-like lesions were induced by exposure for 6 weeks to *Streptococcus mutans* GEM, a biotype I clinical isolate *(S. m.),* and *Lactobacillus casei* (ATCC 11578) (*L. c*.) according to the following protocol: mounted teeth were placed in Brain Heart Infusion broth (BHI; Difco, Detroit, MI) containing 5.0% sucrose w/v which was inoculated with *S*. *m.* The medium was changed daily and incubation conducted at 37°C for 7 days in air containing 10% CO₂. At day 8, mounted teeth were placed in MRS broth (Difco) containing 5.0% sucrose which was inoculated with *L. c*. This medium was alternated daily with a solution of 0.85% NaCl (normal saline) and 5.0% w/v sucrose. Incubation proceeded for an additional 97 days. Incremental caries progression was evaluated by sequential radiography. Spot microbial culturing of lesions was conducted.

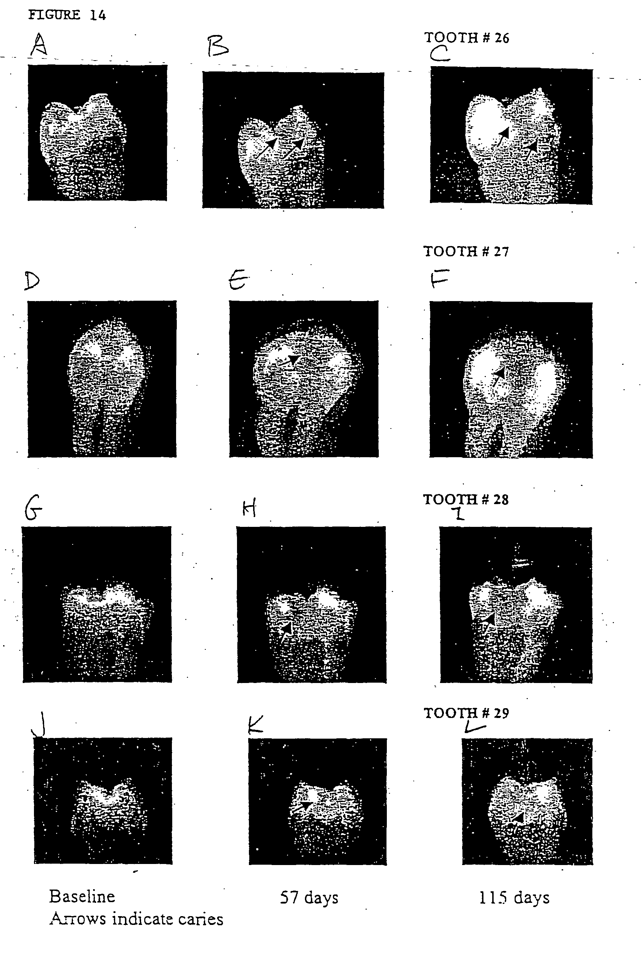

Teeth were incubated with pure cultures of cariogenic bacteria for 105 days. Dentinal-like carious lesions appeared in the teeth and progressed with time (Figures 14A to 14L).

Example 4. Effect of a 1-Minute Rinse with Trim or Water on Acidogenesis of Experimental Dental Plaque (Indirect Plaque pH Measurements) See Figures 2-9.

The purpose of this experiment is to simulate the effect on acidogenesis of plaque following a 1-minute Trim rinse.

Mounted teeth containing cariogenic plaques were removed from the incubation medium and rinsed in 0.85% saline solution (normal saline) to remove substrates and allow glycolysis to cease. Mounted teeth were then placed in 2 ml of water with 5% sucrose for 25 minutes at 37°C. This procedure simulated a typical dietary exposure to a cariogenic substrate. During this time pH of the water was measured by a pH meter (Orion 420A, Fisher Scientific) with a Ross combination microelectrode (Orion, Fisher). Teeth were then immersed for 1 min in either Trim (test solution) or water (control solution) after which they were placed in 2 ml of water and the pH was monitored for additional 25 minutes.

Teeth exposed to 5% sucrose in water for 25 minutes decreased the plaque pH from approximately 6.5 to 4.00. After a 1 minute water rinse the pH returned to about 5.00 and dropped to 4.00 after an additional 25 minutes of incubation.

After a 1 minute Trim rinse, the pH rose to roughly 7.5 and then dropped to about 5.5 after an additional 25 minute incubation.

Trim appeared to have a protective effect on cariogenic plaque after a 1 minute rinse since acidity was immediately raised to a safe level and it remained above the critical pH of 5.5 for up to 25 minutes.

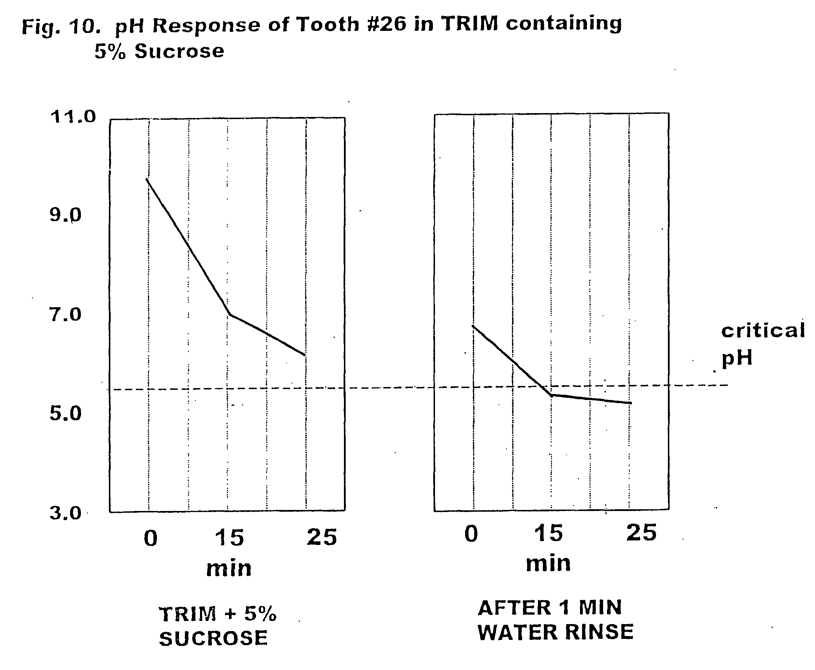

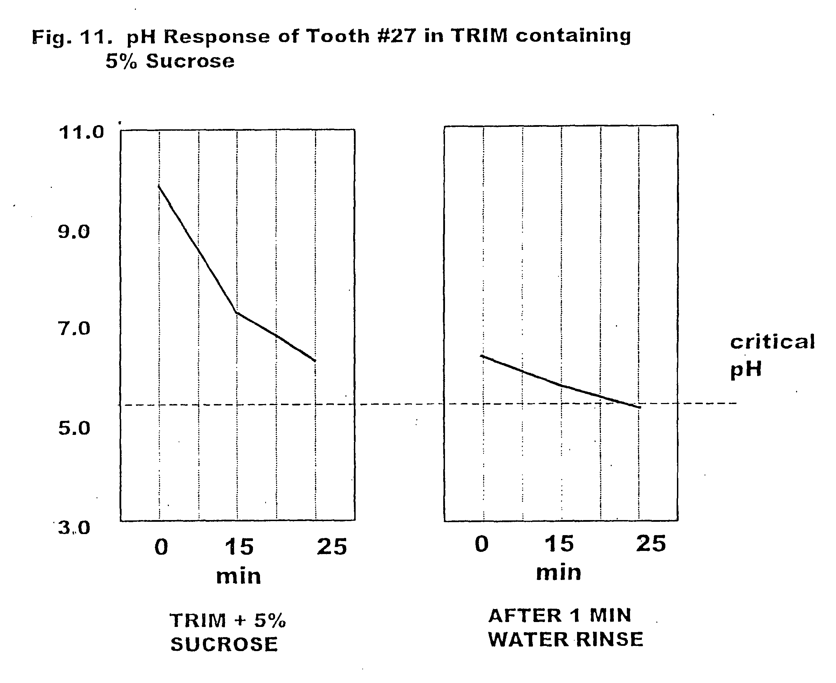

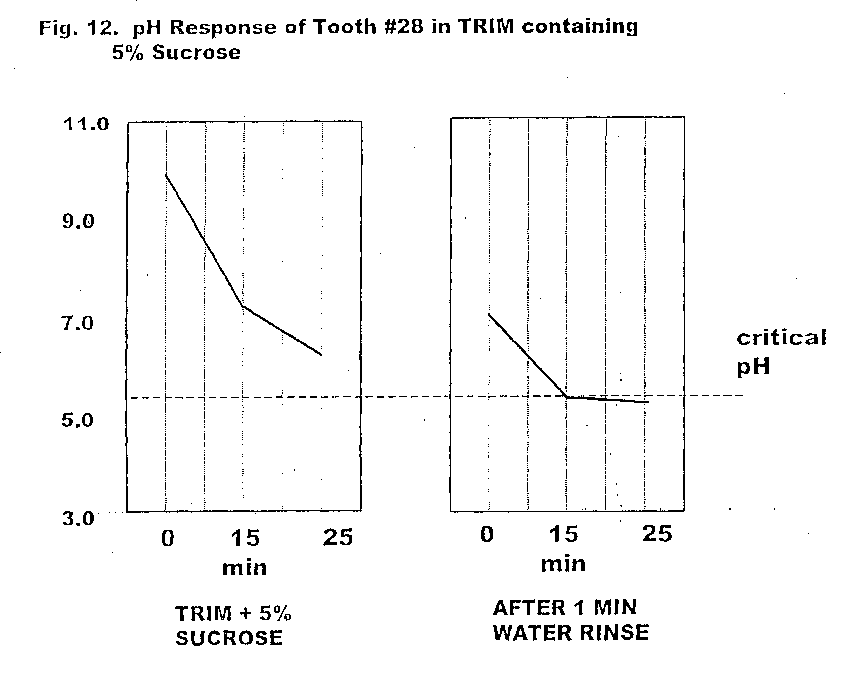

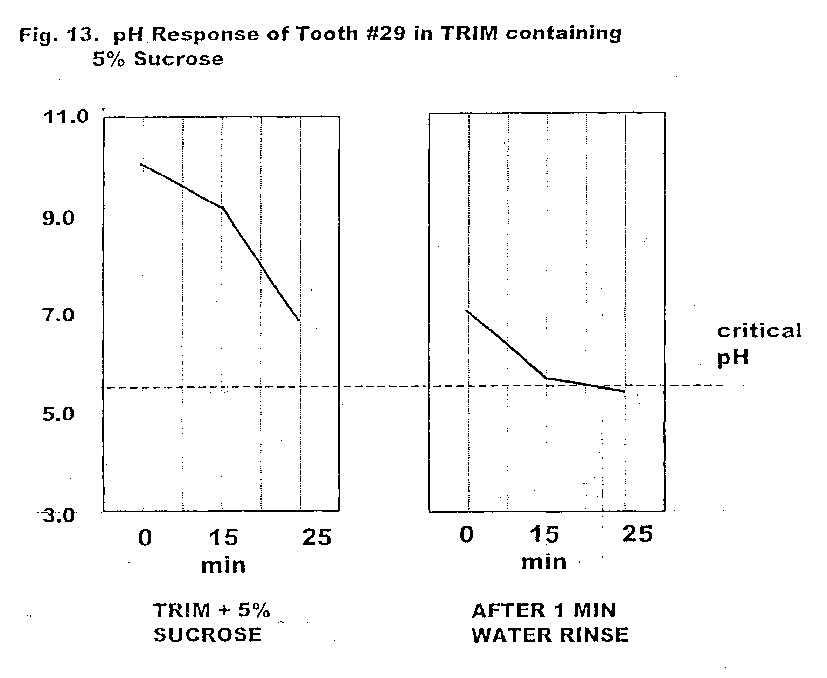

Example 5. Effect of Trim or Water containing 5% sucrose on Acidogenesis of Experimental Cariogenic Plaque (Indirect Plaque pH Measurements). See Figures 10-13.

The purpose of this experiment is to test plaque acidogenesis during exposure to Trim containing a cariogenic substrate.

The steps outlined in Example 2 above were repeated with the exception that Trim containing 5% sucrose replaced 5% sucrose in water during the first incubation. All teeth were then rinsed in water for 1 minute and the pH was followed for additional 25 minutes.

When Trim containing 5% sucrose was incubated with cariogenic plaques for 25 minutes the pH decreased from about 10.0 to 6.2. After a 1 minute water rinse the pH continued to drop to approximately 5.0 when incubated in water for additional 25 minutes.

These results indicate that if Trim were the base for a sweet drink, for example, pH of plaque would not decrease to a cariogenic level when the Trim drink is present in the mouth. Plaque acidogenesis would continue, however, after it is cleared by washing with water or perhaps, swallowing.

### Example 6. Effect of a 1-minute rinse with Trim or Water on Acidogenesis of Experimental Dental Plaque (Direct Plaque pH Measurements)

The purpose of this experiment is to simulate the effect on acidogenesis of plaque following a 1-minute Trim rinse.

The steps outlined in Example 2 above was followed with the exception that plaque pH was measured directly with a microelectrode (Wpi Inc. MEPH-3 touch microelectrode, Londonderry, NH).

In this experiment, pH was measured directly within experimental lesions. During a 25-minute incubation with 5% sucrose, the pH decreased only slightly from approximately 4.2 to 4.0. After a 1 minute rinse with Trim the pH rose to roughly 7.00 in 3 of 4 teeth and decreased to about 4.5 during a second 25 minute incubation. When the plaques were rinsed with the control solution, water, and then incubated for additional 25 minutes, the pH remained at approximately 4.00. This experiment seemed to indicate that Trim buffering capacity would not be very effective in deep carious lesion. Results are tabulated below (Table 2).

**Table 2. Effect of a 1-Minute Trim or Water Rinse on Acidogenesis of Artificial Carious Lesions**

| Incubations | #26 | #27 | #28 | #29 | Incubations | #26 | #27 | #28 | #29 |
|---|---|---|---|---|---|---|---|---|---|
| Original pH | 3.91 | 3.85 | 4.04 | 4.10 | Original pH | 3.91 | 3.57 | 3.75 | 3.42 |

| CONTROL | | | | | TEST | | | | |
|---|---|---|---|---|---|---|---|---|---|
| Water+5% | | | | | Water+5% | | | | |
| | | | | | | | | | |
| sucrose | | | | | sucrose | | | | |
| 0 min | 4.04 | 3.97 | 4.05 | 4.32 | 0 min | 4.10 | 4.41 | 4.29 | 4.20 |
| 12 min | 4.02 | 4.03 | 4.10 | 4.12 | 12 min | 4.03 | 4.05 | 4.20 | 4.28 |
| 25 min | 4.03 | 4.03 | 4.14 | 4.10 | 25 min | 3.93 | 4.10 | 4.17 | 3.97 |

| 1 min WATER | | | | | 1 min TRIM | | | | |
|---|---|---|---|---|---|---|---|---|---|
| Rinse | | | | | Rinse | | | | |
| Water | | | | | Water | | | | |
| 0 min | 3.92 | 3.88 | 4.23 | 4.13 | 0 min | 6.75 | 4.72 | 7.25 | 7.19 |
| 12 min | 3.94 | 3.98 | 4.19 | 4.15 | 12 min | 4.83 | 4.58 | 4.53 | 5.27 |
| 25 min | 4.06 | 3.99 | 4.11 | 4.03 | 25 min | 4.65 | 4.26 | 4.34 | 4.72 |

## Claims

1. A mouthwash formulation to be administered to teeth, said mouthwash comprising electrolysed water, wherein the electrolysed water has an oxidation-reduction potential within the range - 150mV - 0mV measured against a platinum electrode, wherein the formulation comprises sucrose.

2. The formulation of claim 1, wherein the formulation increases the pH of dental plaque in contact with the formulation to greater than 5.5.

## Patentansprüche

1. mundwasserformulierung zur verabreichung an Zähne, enthaltend elektrolysiertes Wasser, wobei das elektrolysierte Wasser ein gegen eine Platinelektrode gemessenes Oxidations-ReduktionsPotential im Bereich von -150 mV - 0 mV aufweist, wobei die Formulierung Saccharose enthält.

2. Formulierung nach Anspruch 1, wobei die Formulierung den pH-Wert von Zahnbelag in Kontakt mit der Formulierung auf mehr als 5,5 erhöht.

## Revendications

1. Formulation de rince-bouche destinée à être administrée à des dents, ledit rince-bouche comprenant de l'eau électrolysée, où l'eau électrolysée présente un potentiel d'oxydation-réduction dans la plage - 150 mV - 0 mV, mesuré par rapport à une électrode de platine, où la formulation comprend du sucrose.

2. Formulation selon la revendication 1, où la formulation accroît le pH de la plaque dentaire en contact avec la formulation à plus de 5,5.
